# EUROPÄISCHE PATENTSCHRIFT

(11) **EP 1 637 098 B1**
(45) Veröffentlichungstag und Bekanntmachung des Hinweises auf die Patenterteilung: **18.11.2009**
(21) Anmeldenummer: 05026758.2
(22) Anmeldetag: 23.04.2002
(51) Int. Cl.: A61F 5/01

(54) **Unterschenkelorthese**
Lower leg orthosis
Orthese de jambe

(30) Priorität: 31.05.2001 DE 10126622
(43) Veröffentlichungstag der Anmeldung: 22.03.2006
(62) Teilanmeldung aus: 02737814.0
(73) Patentinhaber: Gottinger Handelshaus GbR, 85604 Zorneding (DE)
(72) Erfinder: Günther, Norbert G., 85599 Parsdorf (DE)
(74) Vertreter: Winter, Brandl, Fürniss, Hübner Röss, Kaiser, Polte Partnerschaft Patent- und Rechtsanwaltskanzlei

(56) Entgegenhaltungen:
- DE-U- 29 908 981

## Beschreibung

Die Erfindung betrifft eine Unterschenkelorthese gemäß dem Oberbegriff des Patentanspruchs 1, wie sie aus der DE 299 08 981 U1 bekannt ist und eine dafür vorgesehene Stützfeder.

Derartige Orthesen werden beispielsweise bei Patienten mit einer tiefen Lähmung, bei Muskelerkrankungen, infantilen Zerebralparesen, pathologischen Veränderungen, neurologischen Erkrankungen oder auch bei gesunden Menschen eingesetzt, um die Funktion des Menschenfußes zu unterstützen. Durch die Orthese wird der Fuß mit Bezug zum Unterschenkel gehalten, wobei eine Bewegung nach vorn und hinten, d. h. in Längsrichtung bestimmbar freigegeben wird. Dabei ist die Beweglichkeit nach vorne in der Regel über einen größeren Bereich als die Beweglichkeit nach hinten ausgebildet. Die Relativbeweglichkeit des Fußes in Querrichtung mit Bezug zum Unterschenkel soll in den meisten Fällen auf ein Minimum reduziert sein.

Die bisher verwendeten Orthesen haben eine Fußmanschette und eine Unterschenkelmanschette, die über Knöchelgelenke aus Metall miteinander verbunden sind. Nachteilig bei dieser Konstruktion ist, dass die Knöchelgelenke einer erheblichen Belastung und damit einem erheblichen Verschleiß unterworfen sind und daher vergleichsweise stabil ausgeführt sein müssen. Die Beweglichkeit wird dabei durch Anschläge vorgegeben, die im Knöchelgelenk ausgebildet sind.

Aufgrund der vergleichsweise stabilen Ausgestaltung der Knöchelgelenke haben diese bekannten Orthesen ein erhebliches Gewicht, das die Beweglichkeit der Patienten beeinträchtigt. Nachteilig ist desweiteren, dass stabile Knöchelgelenke einen erheblichen Bauraum erfordern, so dass die Orthese relativ plump wirkt.

Gemäß dem Gebrauchsmuster 299 08 981.9 ist eine Unterschenkelorthese mit einer Stützfeder in einer einstückig ausgebildeten Unterschenkelmanschette und Fußmanschette bekannt, bei der im Übergangsbereich ein Gelenkschlitz ausgebildet ist.

Nachteilig an diesem Gebrauchsmuster ist jedoch, dass durch die einstückige Ausprägung leichte Pendelbewegungen des Patienten zur ungewollten Schritteinleitung führen, wodurch erhebliche Gleichgewichtsprobleme entstehen.

Die Aufgabe der Erfindung besteht darin, eine Unterschenkelorthese und eine Stützfeder zu schaffen, durch die eine hinreichende Beweglichkeit und Stabilität mit minimalem vorrichtungstechnischen Aufwand gewährleistet ist.

Erfindungsgemäß hat die Unterschenkelorthese zumindest eine Stützfeder, über die eine höhenverstellbare Unterschenkelmanschette und eine Fußmanschette miteinander verbunden sind. Diese Stützfeder wird derart ausgelegt, dass sie die Beweglichkeit in Längsrichtung (Fuß anheben, Fuß absenken) im erforderten Bereich freigibt. Die Stützfeder kann so ausgelegt sein, dass die Beweglichkeit in Querrichtung minimal (steif) ist oder derart, dass eine gewollte Flexibilität zugelassen wird, um eine gewollte Gegenrotation und Lenkung zu ermöglichen.

Die Stützfeder speichert bei der Bewegung, beispielsweise beim Abwinkeln des Fußes potentielle Energie, so dass die Dorsalflexion während der Schwungphase durch die Entspannung der Stützfeder ermöglicht und unterstützt wird. Auf diese Weise ist ein energiesparendes Gehen mit gegenüber herkömmlichen Lösungen verringertem Kraftaufwand möglich.

Die Stützfeder ermöglicht des Weiteren ein Bergauf- und Bergabgehen durch Schwerpunktverlagerung.

Bei einem besonders bevorzugten Ausführungsbeispiel ist die Stützfeder als etwa L-förmige Blattfeder ausgebildet mit im Fersenbereich vorzugsweise zwei gegenläufig gekrümmten Abschnitten, die in einen fußseitigen und einen unterschenkelseitigen Endabschnitt übergehen. Die Blattfeder erstreckt sich entlang der Rückseite des Unterschenkels über die Ferse und entlang eines Teils der Fußmanschettensohle. Die Stützfeder kann dabei in vorgesehene Aufnahmen aufgenommen oder in das Manschettenmaterial eingebettet werden.

Die Blattfeder ist dabei derart in der Fußmanschette geführt, dass sie nur an ihrem fußseitigen Endabschnitt mit der Fußmanschette verbunden ist und sich zwischen der hinteren Stirnseite der Fußmanschette und dem Fersenteil bzw. dem unterschenkelseitigen Teil der Blattfeder ein Spalt ergibt. Über einen definierten Anschlag an der hinteren Seite der Fußmanschnette wird die Relativbewegung der Fußmanschette bzgl. dem Fersenteil der Stützfeder bei der Schritteinleitung begrenzt.

Um einigen Patienten über die Rotation der Stützfeder um die vertikale Längsachse eine höhere Bewegungsfreiheit zu geben, bspw. mittels einer Reduzierung der Stützfederbreite im Mittelteil (Fersenteil), aber dennoch den Fuß in einer für den Patienten optimalen Art bei der Schritteinleitung zu führen, sieht eine Ausführungsform eine vorzugsweise keilförmige Ausprägung des Anschlags vor.

Eine Alternative zu dem keilförmigen Anschlag ist eine Eingriffvorrichtung in Höhe des Fersenteils, bei der z. B. ein Keil an der Fußmanschette in eine Nut in der Stützfeder greift.

Eine Unterschenkelorthese mit besonders hoher Stabilität und geringem Gewicht erhält man, wenn die Stützfeder aus faserverstärktem Kunststoff, vorzugsweise aus Kohlefaser hergestellt wird.

Sonstige vorteilhafte Weiterbildungen der Erfindung sind Gegenstand der weiteren Unteransprüche.

Im Folgenden wird ein bevorzugtes Ausführungsbeispiel der Erfindung anhand schematischer Zeichnungen näher erläutert. Es zeigen:
Figur 1 eine vereinfachte Darstellung einer erfindungsgemäßen Unterschenkelorthese in Seiten- und Rückansicht;
Figur 2 einen Schnitt entlang der Linie A-A in Figur 1;
Figur 3 eine schematische Darstellung der Ruheposition und direkt nach der Schritteinleitung;
Figur 4 eine schematische Zeichnung einer Stützfeder in Seitenansicht;
Figur 5 eine schematische Zeichnung einer Stützfeder in Rückansicht;
Figur 6 eine vereinfachte Draufsicht auf einen Anschlag in Keilform und
Figur 7 eine schematische Zeichnung einer Eingriffseinrichtung in Nut-Feder-Ausprägung.

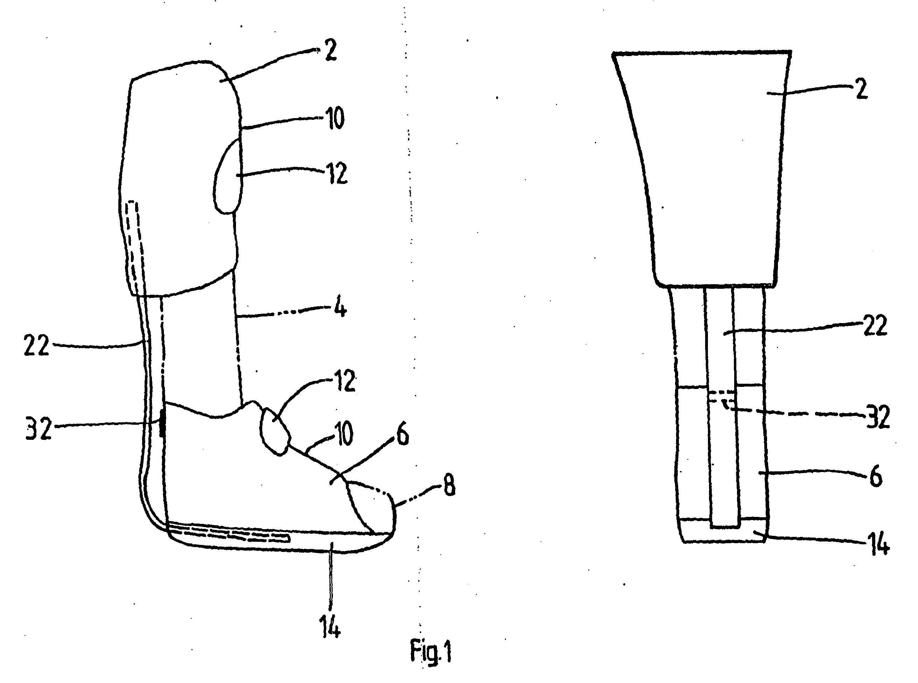

Figur 1 zeigt eine schematische Darstellung einer Unterschenkelorthese 1, die bei tiefen Lähmungen zum Stützen des Fußes verwendet wird. Durch die Orthese soll die Funktion des oberen Sprunggelenks, d. h. das Anheben und Absenken des Fußes in Längsrichtung (nach vorn und nach hinten) ermöglicht werden. Die Funktion des unteren Sprunggelenkes, d. h. das Ermöglichen eines seitlichen Anhebens und Absenkens soll durch die erfindungsgemäße Unterschenkelorthese nicht ermöglicht werden, d. h. die Unterschenkelorthese 1 ist in der Bewegungsrichtung senkrecht zur Zeichenebene der Figur 1 vergleichsweise steif ausgelegt.

Die Unterschenkelorthese 1 umgreift mit einer Unterschenkelmanschette 2 den Unterschenkel 4 (dreifach gepunktet angedeutet) und mit einer Fußmanschette 6 den Fuß 8 (zweifach gepunktet) des Patienten, wobei diese über eine die Bewegung steuernde Stützfeder 22 miteinander verbunden sind.

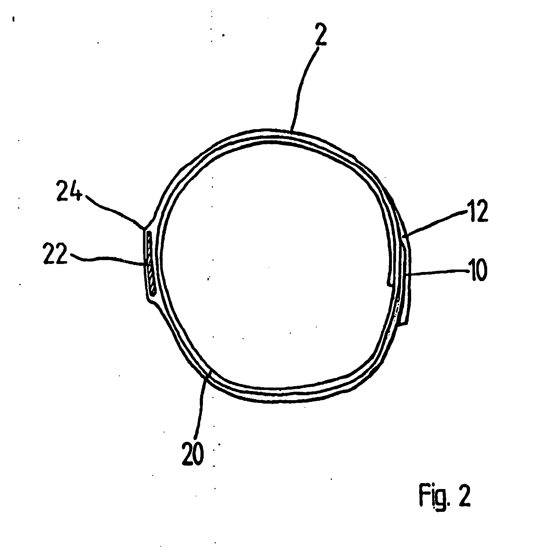

Die Manschetten (2; 6) dienen zur Übertragung der Stützkräfte von der Stützfeder 22 auf das Bein und sind vorzugsweise aus Kunststoff hergestellt. Zur individuellen Anpassung an die Unterschenkellänge eines jeden Patienten ist die Unterschenkelmanschette 6 höhenverstellbar. Gemäß Figur 2 haben die Manschetten (2; 6) an ihrer vorderen Stirnseite eine Überlappung 10, die zum Ansetzen der Unterschenkelorthese 1 geöffnet werden kann, um den Unterschenkel in die Unterschenkelmanschette 2 oder den Fuß 8 in die Fußmanschette 6 einzuführen. Die beiden die Überlappung 10 definierenden Endabschnitte jeder Manschette (2; 6) werden durch einen geeigneten Verschluss, beispielsweise einen Klettverschluss 12 lagefixiert, der die beiden Endabschnitte der Überlappung 10 überstreckt. Zur Verminderung der Abnutzung ist die Fußmanschette 6 mit einer Sohle 14 versehen. Zur Erhöhung des Tragekomforts sind die Manschetten (2; 6) mit einer Polsterung 20 versehen, die beispielsweise den Umfangsrand umgreifen kann.

Die Führung des unterschenkelseitigen Endabschnittes 26 an bzw. in der Unterschenkelmanschette 2 kann beispielsweise erfolgen, indem an die hintere Stirnfläche der Unterschenkelorthese 1 eine Rippe 24 angesetzt wird, die die Stützfeder 22 umgibt. Alternativ könnte die Stützfeder 22 auch in einer geeigneten Aufnahme angeordnet werden, die an der Unterschenkelorthese 1 ausgebildet ist. Prinzipiell vorstellbar ist auch eine Befestigung auf andere Weise, beispielsweise durch Schrauben oder Nieten auf den Außenumfang der Unterschenkelmanschette 6, wobei in diesem Fall eine Einlegenut zur Aufnahme der Stützfeder 22 ausgebildet werden sollte. Alternativ könnte die Stützfeder 22 mit der Unterschenkelmanschette 2 auch direkt an einem Schuh befestigt werden.

Die Führung der Stützfeder 22 in der Fußmanschette 6 erfolgt erfindungsgemäß derart, dass zwischen der hinteren Stirnseite der Fußmanschette 6 und dem Fersenteil 30 bzw. einem unterschenkelseitigen Stützfederteil im Ruhezustand, d. h. im Stand, keine Berührung stattfindet. Die Verbindung zwischen der Stützfeder 22 und der Fußmanschette 6 erfolgt über den fußseitigen Endabschnitt 28, der vorzugsweise in die Sohle 14 eingebettet ist.

Über einen definierten Anschlag 32 an der hinteren Stirnseite der Fußmanschette 6 wird die Biegung der Stützfeder 30 in Richtung der Fußmanschette 6 bei der Schritteinleitung begrenzt, wodurch eine Stabilisierung der Schritteinleitung erzielt wird. Der Anschlag 32 kann lösbar ausgebildet sein und sitzt dann vorzugsweise in einer Art Tasche. Die Befestigung der Tasche kann sowohl an der hinteren Stirnseite der Fußmanschette 6 als auch an der Stützfeder 22 erfolgen. Eine Befestigung des Anschlags 32 mittels einer Klemm- oder Schraubverbindung ist ebenfalls vorstellbar.

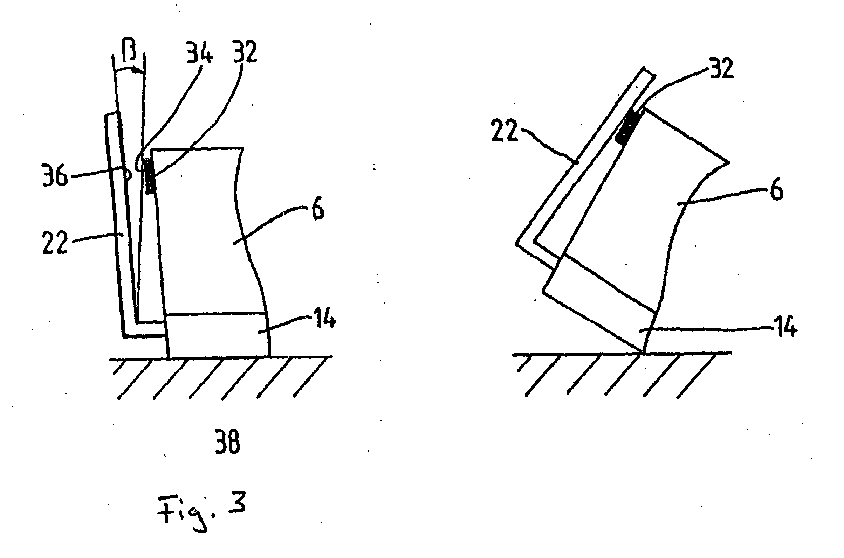

Bei einer bevorzugten Anwendungsform gemäß Figur 3 läuft die Stützfeder 22 ab einem Winkel β ≥ 5° auf die Anschlagsfläche 34 auf. Erfindungsgemäß wird dem Patienten so die Möglichkeit geschaffen, in der Ruheposition 38 Pendelbewegungen in Gehrichtung von < 5° auszuführen, ohne automatisch die Schritteinleitung zu beginnen. Erst ab Pendelwegungen ≥ 5° wird die Sohle 14 der Fußmanschette 6 über die auf den Anschlag 32 auflaufende Stützfeder 22 von dem Boden abgehoben und somit der Gehprozess eingeleitet.

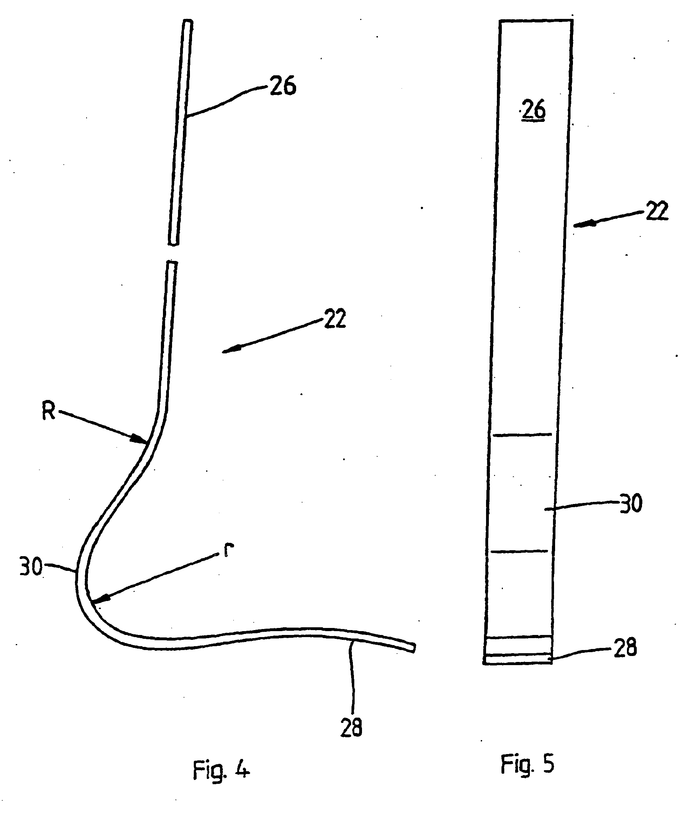

Die Stützfeder 22 gemäß Figur 4 hat einen etwa L-förmigen Aufbau mit einem unterschenkelseitigen Endabschnitt 26 und einem fußseitigen Endabschnitt 28, die von einem gegenläufig gekrümmten Fersenteil 30 miteinander verbunden sind. Die Krümmung des Fersenteils 30 wird durch zwei Radien R und r ausgebildet. Der Radius r und der sich bis zur Unterschenkelmanschette erstreckende Teil der Stützfeder 22 bestimmen im Wesentlichen die Beweglichkeit der Stützfeder 22 nach vorne (rechts in Figur 3), während der Radius R die Beweglichkeit der Stützfeder 22 nach hinten (links in Figur 3) überwiegend bestimmt. Das heißt, durch geeignete Abstimmung der Radien R und r sowie der Länge der gegenläufig gekrümmten Abschnitte kann das Biegeverhalten relativ einfach an die Bedürfnisse des jeweiligen Patienten angepasst werden. In der Regel wird man die Stützfeder 22 so auslegen, dass eine Bewegung nach hinten nur in eingeschränktem Umfang möglich ist, während die Bewegung nach vorne über einen größeren Winkelbereich möglich sein soll. Die Beweglichkeit wird dabei in den Bereich in der Nähe des oberen Sprunggelenks gelegt.

Anstelle einer Stützfeder könnten auch mehrere Federn paralell angeordnet werden, so dass sich unterschiedliche Federraten einstellen lassen.

Wie aus der Darstellung gemäß Figur 5 hervorgeht, ist die Stützfeder 22 als Blattfeder ausgebildet, wobei als Material vorzugsweise ein faserverstärkter Kunststoff, beispielsweise kohlenfaserverstärkter Kunststoff verwendet wird. Dieses Material zeichnet sich durch eine hervorragende Biegesteifigkeit bei minimalem Gewicht und hoher Dauerfestigkeit aus. Prinzipiell lassen sich jedoch auch andere geeignete Materialien einsetzen, die jedoch stets im Hinblick auf minimales Gewicht und maximale Dauerfestigkeit auszuwählen sind.

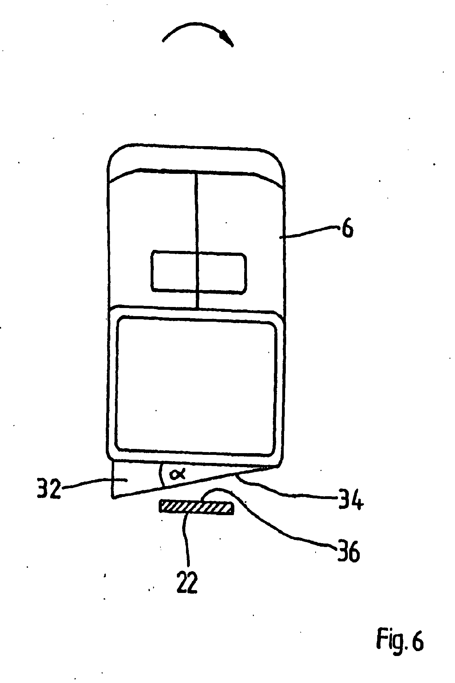

Figur 6 zeigt eine Ausführungsform des Anschlags 32, in der der abnehmbare Anschlag 32 keilförmig ausgeprägt ist, wobei die Neigung der Anschlagfläche 34 derart verläuft, dass der Fuß 8 bei der Schritteinleitung eine Rotation um die vertikale Achse der Unterschenkelorthese 1 in Gehrichtung im Uhrzeigersinn ausführt. Dabei lässt sich sowohl der Rotationswinkel als auch die Rotationsrichtung nur über die Neigung und Ausrichtung des Keilwinkels γ einstellen. Durch den Einsatz des Anschlags 32 in erfindungsgemäßer keilförmiger Ausprägung wird verhindert, dass der Fuß bei der Schritteinleitung seitlich abknickt, was besonders häufig bei gelähmten Patienten zu beobachten ist.

Des Weiteren kann bestimmten Patienten zusätzlich zu der Pendelbewegung von ≤ 5° in Gehrichtung mehr Bewegungsfreiheit in vertikaler Rotationsrichtung in der Ruheposition 38, z. B. über eine Verjüngung der Stützfeder im Fersenteil 30, gegeben werden, ohne jedoch bei der Schritteinleitung auf die notwendige Stabilisierung und Führung des Fußes zu verzichten. Das heißt, eine derartige Unterschenkelorthese ermöglicht eine hohe Anzahl von Freiheitsgraden, wodurch der Tragekomfort wesentlich erhöht wird.

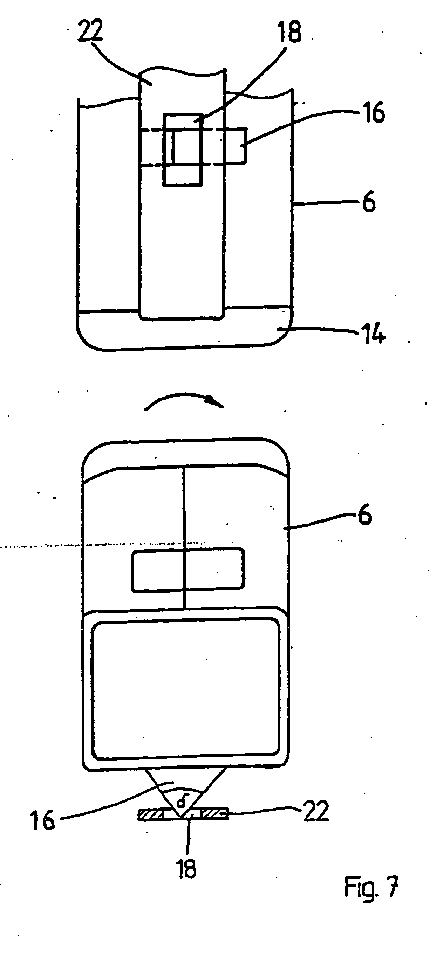

Eine Alternative zu dem keilförmigen Anschlag 32 ist in Figur 7 dargestellt. Ein Keil 16 an der hinteren Stirnseite der Fußmanschette 6 greift in eine Nut 18 der Stützfeder 22 im Fersenteil 30 ein. Der Biegungsgrad der Stützfeder 22 und die Rotationsrichtung kann dabei über den Neigungswinkel δ und die Symmetrieeigenschaften des Keils 16 definiert werden.

Selbstverständlich ist die Geometrie der Stützfeder 22 nicht auf das in Figur 4 dargestellte Rechteckprofil beschränkt, sondern es könnten auch andere, über die Länge der Stützfeder 22 variierende Profile eingesetzt werden um die Beweglichkeit für eine Rotation, eine Dorsalflexion und eine Plantarflexion zur Verfügung zu stellen.

Die erfindungsgemäße Verwendung einer Stützfeder 22 ermöglicht es, das Bein während des Gehens in seine Ruhelage zurückzubringen, so dass der individuelle Schwerpunkt in der Standphase auch bei der Lähmung von Muskulaturbereichen, bei Wahrnehmungsproblemen und auch bei gesunden Personen ins Körperlot gebracht wird. Die Stützfeder 22 wird so angebracht, dass einerseits durch hinreichende Stabilität die Standsicherheit erhöht wird, andererseits durch eine ausreichende Flexibilität (Größe und Form der Feder) eine Gangsicherung bewirkt wird.

Offenbart ist eine Unterschenkelorthese, bei der eine Dorsalflexion und eine Plantarflexion durch eine Stützfeder ermöglicht wird, die eine Unterschenkelmanschette und eine Fußmanschette verbindet. Die Stützfeder ist derart in die Fußmanschette eingespannt, dass sich zwischen dem Fersenteil und der Fußmanschette ein Spalt ergibt, wobei die Biegung der Stützfeder in Schrittrichtung über einen Anschlag an der Fußmanschette begrenzt ist.

### Bezugszeichenliste:

- 1: Unterschenkelorthese
- 2: Unterschenkelmanschette
- 4: Bein
- 6: Fußmanschette
- 8: Fuß
- 10: Überlappung
- 12: Klettverschluss
- 14: Sohle
- 16: Keil
- 18: Nut
- 20: Polsterung
- 22: Stützfeder
- 24: Rippe
- 26: unterschenkelseitiger Endabschnitt
- 28: fußseitiger Endabschnitt
- 30: Fersenteil
- 32: Anschlag
- 34: Anschlagfläche
- 36: auflaufende Fläche
- 38: Ruheposition

## Patentansprüche

1. Unterschenkelorthese mit einer den Unterschenkel (4) umgreifenden Unterschenkelmanschette (2), die zumindest mit einer Stützfeder (22) gelenkig mit einer Fußmanschette (6) verbunden ist, **gekennzeichnet durch** einen Anschlag (32), der die Biegung der Stützfeder (22) in Richtung der Fußmanschette (6) begrenzt.

2. Unterschenkelorthese nach Anspruch 1, **dadurch gekennzeichnet, dass** der Anschlag (32) mit der hinteren Stirnseite der Fußmanschette (6) oder an der Stützfeder (22) lösbar befestigt ist.

3. Unterschenkelorthese nach den Ansprüchen 1 und 2, **dadurch gekennzeichnet, dass** zwischen einem Fersenteil (30) oder einem unterschenkelseitigen Teil der Stützfeder (22) und der hinteren Stirnseite der Fußmanschette (6) in der Ruheposition (38) keine Berührung stattfindet.

4. Unterschenkelorthese nach einem der vorhergehenden Ansprüche, **dadurch gekennzeichnet, dass** die Anschlagfläche (34) gegenüber der auf diese auflaufende Stützfläche (36) parallel oder schräg angestellt ist.

5. Unterschenkelorthese nach einem der vorhergehenden Ansprüche, **dadurch gekennzeichent, dass** der Anschlag (32) ein Keil ist.

6. Unterschenkelorthese nach einem der vorhergehenden Ansprüche, **dadurch gekennzeichnet, dass** die Stützfeder (22) ab einem Winkel β ≥ 5° den Anschlag berührt.

7. Unterschenkelorthese nach einem der vorhergehenden Ansprüche, **dadurch gekennzeichnet, dass** die Stützfeder (22) in einem Fersenteil (30) vorzugsweise gegenläufig gekrümmt ist und die beiden Endabschnitte (26, 28) die Unterschenkelrückseite bzw. die Fußsohle in Längs- und/oder Querrichtung abstützen.

8. Unterschenkelorthese nach einem der vorhergehenden Ansprüche, **dadurch gekennzeichnet, dass** die Stützfeder (22) mit der Fußmanschette (6) über den fußseitigen Endabschnitt (28) verbunden ist.

9. Unterschenkelorthese nach einem der vorhergehenden Ansprüche, **dadurch gekennzeichnet, dass** die Unterschenkelmanschette (2) höhenverstellbar ist.

10. Unterschenkelorthese nach einem der vorhergehenden Ansprüche, **dadurch gekennzeichnet, dass** die Stützfeder eine Blattfeder (22) ist.

11. Unterschenkelorthese nach einem der vorhergehenden Ansprüche, **dadurch gekennzeichnet, dass** die Blattfeder (22) aus faserverstärktem Kunststoff, vorzugsweise aus kohlefaserverstärktem Kunststoff hergestellt ist.

12. Unterschenkelorthese nach einem der vorhergehenden Ansprüche, **dadurch gekennzeichnet, dass** eine lösbare Eingriffsvorrichtung, über die die Stützfeder (22) und die Fußmanschette (6) ab einem vorbestimmten Neigungswinkel δ lösbar miteinander verbunden sind.

## Claims

1. A below-knee orthotic device, including a lower leg sleeve (2) enveloping the lower leg (4) and hingedly connected to a foot sleeve (6) by at least one support spring (22), **characterized by** a stop (32) which limits bending of said support spring (22) toward said foot sleeve (6).

2. The below-knee orthotic device according to claim 1, **characterized in that** said stop (32) is releasably secured with the rear face of said foot sleeve (6) or on said support spring (22).

3. The below-knee orthotic device according to claims 1 and 2, **characterized in that** in the position of rest (38), there is no contact between a heel part (30) or a part of said support spring (22) on the lower leg side and the rear face of said foot sleeve (6).

4. The below-knee orthotic device according to any one of the preceding claims, **characterized in that** said stop surface (34) is set to be parallel with, or inclined relative to said support surface (36) entering into contact with it.

5. The below-knee orthotic device according to any one of the preceding claims, **characterized in that** said stop (32) is a wedge.

6. The below-knee orthotic device according to any one of the preceding claims, **characterized in that** said support spring (22) contacts said stop starting from an angle β ≥ 5°.

7. The below-knee orthotic device according to any one of the preceding claims, **characterized in that** said support spring (22) preferably has a reversing curvature in a heel part (30), and **in that** said two end portions (26, 28) support the lower leg rear side or the sole of the foot in the longitudinal and/or transverse direction.

8. The below-knee orthotic device according to any one of the preceding claims, **characterized in that** said support spring (22) is connected to said foot sleeve (6) via said end portion (28) on the foot side.

9. The below-knee orthotic device according to any one of the preceding claims, **characterized in that** said lower leg sleeve (2) is adjustable in height.

10. The below-knee orthotic device according to any one of the preceding claims, **characterized in that** said support spring is a leaf spring (22).

11. The below-knee orthotic device according to any one of the preceding claims, **characterized in that** said leaf spring (22) is manufactured of fiber reinforced plastic, preferably of carbon fiber reinforced plastic.

12. The below-knee orthotic device according to any one of the preceding claims, **characterized by** a releasable engagement means whereby said support spring (22) and said foot sleeve (6) are releasably connected from a predetermined inclination angle δ.

## Revendications

1. Orthèse de jambe avec une jambière (2) entourant la jambe (4), reliée au moins avec un ressort de support (22) de manière articulée avec une chevillière (6), **caractérisée par** une butée (32) qui limite le cintrage du ressort de support (22) en direction de la chevillière (6).

2. Orthèse de jambe selon la revendication 1, **caractérisée en ce que** la butée (32) est fixée avec la face arrière de la chevillière (6) ou de manière amovible au ressort de support (22).

3. Orthèse de jambe selon les revendications 1 et 2, **caractérisée en ce qu'**il n'y aucun contact en position de repos (38) entre une partie formant talon (30) ou une partie du côté de la jambe du ressort de support (22) et la face arrière de la chevillière (6).

4. Orthèse de jambe selon l'une des revendications précédentes, **caractérisée en ce que** la surface de butée (34) est placée de manière parallèle ou de biais par rapport à la surface d'appui (36) montant sur celle-ci.

5. Orthèse de jambe selon l'une des revendications précédentes, **caractérisée en ce que** la butée (32) est une clavette.

6. Orthèse de jambe selon l'une des revendications précédentes, **caractérisée en ce que** le ressort de support (22) entre en contact avec la butée à partir d'un angle β ≥ 5°.

7. Orthèse de jambe selon l'une des revendications précédentes, **caractérisée en ce que** le ressort de support (22) est cintré au niveau d'une partie formant talon (30) de préférence de manière opposée et les deux sections d'extrémité (26, 28) supportent la face arrière de la jambe ou la semelle dans le sens de la longueur et/ou dans le sens transversal.

8. Orthèse de jambe selon l'une des revendications précédentes, **caractérisée en ce que** le ressort de support (22) est relié à la chevillière (6) par le biais de la section d'extrémité (28) du côté du pied.

9. Orthèse de jambe selon l'une des revendications précédentes, **caractérisée en ce que** la jambière (2) est réglable en hauteur.

10. Orthèse de jambe selon l'une des revendications précédentes, **caractérisée en ce que** le ressort de support est un ressort à lames (22).

11. Orthèse de jambe selon l'une des revendications précédentes, **caractérisée en ce que** le ressort à lames (22) est fabriqué dans du plastique renforcé par des fibres, de préférence dans du plastique renforcé par des fibres de carbone.

12. Orthèse de jambe selon l'une des revendications précédentes, **caractérisée par** un dispositif de prise amovible, par le biais duquel le ressort de support (22) et la chevillière (6) sont reliés l'un à l'autre de manière amovible à partir d'un angle d'inclinaison prédéterminé δ.
